# EUROPÄISCHE PATENTSCHRIFT

(11) **EP 1 907 346 B1**
(45) Veröffentlichungstag und Bekanntmachung des Hinweises auf die Patenterteilung: **24.12.2008**
(21) Anmeldenummer: 06764183.7
(22) Anmeldetag: 17.07.2006
(51) Int. Cl.: C07C 51/48, C07C 59/08

(54) **VERFAHREN ZUR EXTRAKTION VON MILCHSÄURE AUS WÄSSRIGEN SUSPENSIONEN**
METHOD FOR EXTRACTING LACTIC ACID FROM AQUEOUS SUSPENSIONS
PROCEDE D'EXTRACTION D'ACIDE LACTIQUE DANS DES SUSPENSIONS AQUEUSES

(30) Priorität: 18.07.2005 DE 102005033432
(43) Veröffentlichungstag der Anmeldung: 09.04.2008
(73) Patentinhaber: BASF SE, 67056 Ludwigshafen (DE)
(72) Erfinder: LEBKÜCHER, Helmut, 67433 Neustadt (DE); REISSENWEBER, Gernot, 67459 Böhl-Iggelheim (DE)
(74) Vertreter: Reitstötter - Kinzebach
(86) Internationale Anmeldenummer: PCT/EP2006/064324
(87) Internationale Veröffentlichungsnummer: WO 2007/009970

(56) Entgegenhaltungen:
- EP-A2- 0 159 585
- EP-A2- 0 184 789
- DE-A1- 3 222 837
- DE-B- 1 268 088
- US-A- 2 712 516

## Beschreibung

Die vorliegende Erfindung betrifft ein Verfahren zur kontinuierlichen Extraktion von Milchsäure aus feststoffhaltigen wässrigen Suspensionen durch In-Kontakt-Bringen mit organischen Lösungsmitteln.

Bekanntlich stellt man L(+)-Milchsäure großtechnisch durch fermentativen Abbau glucosehaltiger Rohstoffe her (Ind. Eng. Chem. 44, 1958 (1952)).

Auch die Herstellung von D(-)-Milchsäure durch geeignete Bakterien in ähnlicher Weise ist bekannt (Ullmann's Encyclopedia of Industrial Chemistry, 5. Aufl., Bd. A 15, 97-105, Weinheim, VCH). Hierbei wird die Fermentation in Gegenwart von Calciumcarbonat durchgeführt, so dass man bei der Fermentation zunächst eine wässrige Lösung bzw. Suspension erhält, die üblicherweise 6 bis 20 Gew.-% Calciumlactat enthält. Anschließend wird diese Lösung bzw. Suspension mit Schwefelsäure angesäuert, wobei das Calciumlactat in freie Milchsäure überführt wird und Gips ausfällt. Der hauptsächlich aus Gips und Bakterienmasse bestehende Feststoffanteil der resultierenden Suspension kann bis zu 10 Gew.%, bezogen auf das Gesamtgewicht der Suspension, betragen. Dieser hohe Anteil an Feststoff und insbesondere die auf mechanischem Weg nur schlecht abtrennbare, überwiegend in fein verteilter Form vorliegende Biomasse bereiten bei der Gewinnung reiner Milchsäure aus dem sauren Fermentationsansatz große Schwierigkeiten.

Die äußerst aufwendige und kostenintensive Feststoffabtrennung durch Filtration lässt sich durch das in der EP-A-0 159 585 beschriebene Verfahren zur Extraktion der unfiltrierten, feststoffhaltigen sauren Fermentationsbrühe umgehen. Hierbei wird die Milchsäure durch kontinuierliche Extraktion mit einem organischen Lösungsmittel abgetrennt, indem man die nach Fermentation und Ansäuern erhaltene feststoffhaltige wässrige Suspension in einer mit hydrophoben Einbauten ausgerüsteten Kolonne im Gegenstrom mit dem organischen Lösungsmittel in Berührung bringt. Dies geschieht gemäß der EP-A-0 159 585 besonders zweckmäßig in pulsierten Siebbodenkolonnen und Füllkörperkolonnen mit geordneten Füllkörpern. Hierbei wird die wässrige Suspension (d.h. die angesäuerte Fermentationsbrühe) dem oberen Teil der Kolonne zugeführt und als disperse Phase geführt. Das organische Lösungsmittel wird zur dispersen wässrigen Phase im Gegenstrom geführt. Die Milchsäure wird aus der resultierenden Lösungsmittelphase gewonnen.

Das Extraktionsverfahren der EP-A-0 159 585 lässt sich jedoch nicht in einem großtechnischen Maßstab umsetzen, da Siebbodenkolonnen bereits nach wenigen Stunden Betriebszeit aufgrund des vorhandenen Feststoffanteils zur Verstopfung neigen. Die Durchführung des Verfahrens in einer Füllkörperkolonne gelingt zwar, jedoch haben eigene Versuche der Anmelderin gezeigt, dass hierbei lediglich Extraktionsausbeuten in der Größenordnung von 72 bis 75 % der eingesetzten Milchsäure erzielt werden. Dies führt sowohl zu beträchtlichen Ausbeuteverlusten als auch zu einem mit hoher Fracht an organisch gebundenem Kohlenstoff (TOC = Total organic carbon) belasteten Abwasserstrom.

Es war daher Aufgabe der vorliegenden Erfindung, ein großtechnisch einsetzbares Verfahren zur kontinuierlichen Extraktion von Milchsäure aus feststoffhaltigen wässrigen Suspensionen durch In-Kontakt-Bringen mit organischen Lösungsmitteln bereitzustellen, das eine signifikante Steigerung der Extraktionsausbeute ermöglicht und somit zu einer deutlichen Abnahme des TOC-Gehalts im anfallenden Abwasserstrom führt.

Es wurde nun überraschend gefunden, dass diese Aufgabe gelöst wird, indem man eine Füllkörperkolonne so betreibt, dass im Wesentlichen die organische Phase als disperse Phase geführt wird.

Gegenstand der Erfindung ist daher ein Verfahren zur kontinuierlichen Extraktion von Milchsäure aus einer feststoffhaltigen wässrigen Suspension, bei dem man die feststoffhaltige wässrige Suspension in einer mit Füllkörpern versehenen Kolonne, wobei die Oberflächen der Füllkörper aus hydrophobem Material bestehen, im Gegenstrom mit einem mit Wasser nicht vollständig mischbaren organischen Lösungsmittel unter Ausbildung einer wässrigen und einer organischen Phase so in Kontakt bringt, dass die organische Phase zumindest in einem Abschnitt der Kolonne, der Füllkörper umfasst, als disperse Phase geführt wird.

Das erfindungsgemäße Verfahren ist grundsätzlich zur Extraktion sowohl von D(-)-Milchsäure als auch von L(+)-Milchsäure und deren Gemischen aus feststoffhaltigen Gemischen wie wässrigen Suspensionen geeignet. Erfindungsgemäß werden insbesondere technische wässrige Milchsäuresuspensionen eingesetzt, die auf an sich bekannte Weise durch Ansäuern einer fermentativ hergestellten wässrigen Lösung von Calciumlactat mit Schwefelsäure erhalten werden (Ullmanns Enzyklopädie der technischen Chemie, 3. Aufl., Bd. 12, 525-537, Weinheim, Verlag Chemie). Diese Suspensionen enthalten als Feststoffe insbesondere Biomasse aus der Fermentation und Gips (CaSO₄•2 H₂O). Unter Biomasse wird hier und im Folgenden die Bakterienmasse zusammen mit Resten des verwendeten Nährmediums, z.B. Hefen, verstanden. Suspensionen dieser Art weisen typischerweise einen Gehalt an Milchsäure im Bereich von 5 bis 15 Gew.-% auf. Der Gehalt an Gips liegt typischerweise im Bereich von 5 bis 10 Gew.-%. Der Gehalt an Schwefelsäure liegt üblicherweise im Bereich von 0,1 bis 2 Gew.-%. Der Gehalt an Biomasse liegt im Allgemeinen im Bereich von 0,01 bis 5 Gew.-%. Neben den vorgenannten Komponenten besteht die wässrige Suspension im Wesentlichen aus Wasser. Der Ausdruck "im Wesentlichen" bedeutet hier, dass der Anteil weiterer, von den vorgenannten Komponenten verschiedener Bestandteile in der Regel weniger als 1 Gew.-%, bezogen auf das Gesamtgewicht der Suspension, beträgt.

Erfindungsgemäß werden solche organischen Lösungsmittel eingesetzt, die sich unter den Extraktionsbedingungen, insbesondere bei den angewandten Temperaturen nicht vollständig mit Wasser mischen. Bei dem mit Wasser nicht vollständig mischbaren organischen Lösungsmittel handelt es sich in der Regel um eine aliphatische oder cycloaliphatische, sauerstoffhaltige organische Verbindung mit mindestens 4, z.B. 4 bis 10, häufig 4 bis 8 und insbesondere 4 bis 6 C-Atomen. Üblicherweise weisen die Lösungsmittel keine Heteroatome außer Sauerstoff auf. Zum Beispiel können die Lösungsmittel ein, zwei, drei oder vier Sauerstoffatome je Molekül aufweisen.

Erfindungsgemäß einsetzbare Lösungsmittel sind insbesondere Alkanole mit mindestens 4, z.B. 4 bis 10, häufig 4 bis 8 und insbesondere 4 bis 6 C-Atomen, z.B. Butanole wie 1-Butanol, 2-Butanol und iso-Butanol, Pentanole wie 1-Pentanol, 2-Pentanol, 3-Pentanol, 2-Methyl-1-butanol, 2-Methyl-2-butanol, 3-Methyl-1-butanol, 3-Methyl-2-butanol, 1,1-Dimethylpropanol, 1,2-Dimethylpropanol, 1-Ethylpropanol, Hexanole wie 1-Hexanol, 2-Hexanol, 3-Hexanol, 2-Methyl-1-pentanol, 3-Methyl-1-pentanol, 4-Methyl-1-pentanol, 2-Methyl-2-pentanol, 3-Methyl-2-pentanol, 4-Methyl-2-pentanol, 2-Methyl-3-pentanol, 3-Methyl-3-pentanol, 1,1-Dimethylbutanol, 1,2-Dimethylbutanol, 1,3-Dimethylbutanol, 2,2-Dimethylbutanol, 2,3-Dimethylbutanol, 2,4-Dimethylbutanol, 3,3-Dimethylbutanol, 2-Ethylbutanol, 3-Ethylbutanol, 1,1,2-Trimethylpropanol, Heptanole wie 1-Heptanol, 2-Heptanol, 3-Heptanol, 4-Heptanol, Octanole wie 1-Octanol, 2-Octanol, 3-Octanol, 4-Octanol, 2-Ethylhexanol, cyclische Alkanole wie Cyclopentanol und Cyclohexanol, die beide gegebenenfalls ein, zwei oder drei Alkylseitenketten mit je 1 bis 3 und insbesondere je 1 C-Atom(en) aufweisen können, wie die cis- und trans-Isomere von 2- und 3-Methylcyclopentanol, 2-, 3- und 4-Methylcyclohexanol und 3,3,5-Trimethylcyclohexanol, Ketone mit mindestens 4, z.B. 4 bis 10, häufig 4 bis 8 und insbesondere 4 bis 6 C-Atomen, z.B. 2-Butanon, 2-Pentanon, 3-Pentanon, 2-Hexanon, 3-Hexanon, Cyclopentanon und Cyclohexanon, Ether mit mindestens 4, z.B. 4 bis 10, häufig 4 bis 8 und insbesondere 4 bis 6 C-Atomen, z.B. Diethylether, Di-n-propylether, Di-iso-propylether, Di-n-butylether, Di-iso-butylether und Methyl-tert-butylether, Ester mit mindestens 4, z.B. 4 bis 10, häufig 4 bis 8 und insbesondere 4 bis 6 C-Atomen, z.B. n-Butylacetat, iso-Butylacetat und Pentylacetat. Neben den reinen Lösungsmitteln lassen sich auch Lösungsmittelgemische einsetzen. Vorzugsweise setzt man Alkanole mit 4 bis 8 und insbesondere 4 bis 6 C-Atomen, besonders bevorzugt Butanole und/oder Pentanole und ganz besonders bevorzugt Isobutanol ein. Vorteilhafterweise setzt man das organische Lösungsmittel in mit Wasser gesättigter Form ein.

Die eingesetzte Lösungsmittelmenge kann die 0,1- bis 10-fache, insbesondere die 0,2- bis 5-fache und speziell die 0,5- bis 3-fache Gewichtsmenge der eingesetzten wässrigen Suspension betragen. Die optimale Menge an Lösungsmittel kann der Fachmann ohne Weiteres in Routineexperimenten ermitteln.

Nach dem erfindungsgemäßen Verfahren wird die wässrige Suspension in einer mit Füllkörpern ausgerüsteten Kolonne im Gegenstrom mit dem Lösungsmittel in Kontakt gebracht. Füllkörperkolonnen sind als solche grundsätzlich geeignet und dem Fachmann bekannt. Üblicherweise haben die eingesetzten Kolonnen einen runden, häufig symmetrischen und meistens kreis- oder ellipsenförmigen Durchmesser bzw. Querschnitt. In industriellen Anlagen kann der Durchmesser der eingesetzten Kolonnen z.B. im Bereich von 0,3 bis 2 m, insbesondere im Bereich von 0,3 bis 1,2 m und speziell im Bereich von 0,4 bis 0,8 m liegen. Solche industriell eingesetzten Kolonnen können z.B. eine Länge im Bereich von 3 bis 25 m und insbesondere im Bereich von 5 bis 20 m aufweisen. Naturgemäß ist die Kolonne stehend angeordnet.

Die Füllkörper bestehen aus einem hydrophoben Material oder die Füllkörper sind mit einer Beschichtung aus einem hydrophoben Material versehen. Geeignete hydrophobe Materialien sind insbesondere hydrophobe Polymere, die bei den angewandten Extraktionstemperaturen weitgehend stabil sind. Hierzu zählen z.B. Polyolefine und halogenierte, insbesondere teilweise oder vollständig fluorierte organische Polymere und speziell chlorierte und/oder fluorierte bzw. perfluorierte Polyalkene. Geeignete Polyolefine sind z.B. Polyethylen (PE) und Polypropylen (PP). Geeignete chlororganische Polymere sind z.B. Polyvinylchlorid (PVC) und Polyvinylidenchlorid (PVDC). Beispiele für fluororganische Polymere sind Homo- und Copolymere monoethylenisch ungesättigter fluorierter Monomere wie Chlortrifluorethylen, Hexafluorisobutylen, Hexafluorpropylen, Perfluorvinylmethylether, Tetrafluorethylen, Vinylfluorid und Vinylidenfluorid, speziell Fluor-Thermoplaste wie Poly(vinylfluorid) (PVF), Polyvinylidenfluorid (PVDF), Poly(tetrafluorethylen) (PTFE), Poly(chlortrifluorethylen) (PCTFE), Ethylen/Tetrafluorethylen-Copolymere (E/TFE), Poly(tetrafluorethylen-co-hexafluorpropylen) (FEP), Poly(tetrafluorethylen-co-perfluoralkylvinylether) (PFA/TFA, auch als Perfluoralkoxy-Copolymere bezeichnet), worin die Alkylgruppe z.B. 1 bis 4 C-Atome aufweist, und Fluor-Elastomere wie Hexafluorpropylen/Vinylidenfluorid-Lastomer (CFM), Vinylidenfluorid/Hexafluorpropylen/ Tetrafluorethylen-Copolymer, Tetrafluorethylen/Propylen-Copolymer (TFE/P), Polyfluorsilicone, Polyfluoralkoxyphosphazene und Vulkanisationsprodukte von Fluor-Kautschuken.

Hydrophobe Materialien auf Basis fluorierter organischer Polymere zeichnen sich durch den Vorteil aus, dass sie ein Anhaften von organischen Feststoffen, z.B. Biomasse aus der Fermentation, noch stärker unterdrücken als andere hydrophobe Materialien. In einer bevorzugten Ausführungsform setzt man daher Füllkörper ein, deren Oberflächen von fluororganischen Polymeren gebildet werden. Besonders bevorzugt setzt man Füllkörper ein, die aus Polytetrafluorethylen (PTFE), Polyvinylidenfluorid (PVDF) oder Poly(tetrafluorethylen-co-perfluormethylvinylether), Poly(tetrafluorethylen-co-perfluorethylvinylether), Poly(tetrafluorethylen-co-perftuorpropylvinylether), Poly(tetrafluorethylen-co-perfluorbutylvinylether) (PFA/TFA) oder einer Mischung davon bestehen oder damit beschichtet sind. Es können insbesondere Füllkörper aus Stahl, Glas oder Keramik eingesetzt werden, die mit den vorgenannten hydrophoben Materialien beschichtet wurden. Die Füllkörper können auch vollständig aus dem hydrophoben Material bestehen. Geeignet sind auch Füllkörper aus Kunststoff, beispielsweise Polyethylen, Polystyrol oder Polypropylen, dessen Oberfläche eine fluororganische Beschichtung aufweist.

Bei den Füllkörpern handelt es sich vorteilhafterweise um totraumarme Füllkörper mit durchbrochener Oberfläche. Beispiele für totraumarme Füllkörper mit durchbrochener Oberfläche sind insbesondere zylindrische Typen wie Pallringe, einschließlich modifizierter Pallringe wie die Raflux-Type der Fa. Rauschert, Hiflow-Ringe, Ralu-Ringe der Fa. Raschig, weiterhin Super-Ringe (der Fa. Raschig), sowie kugelförmige Körper mit durchbrochener Kugeloberfläche wie Hacketten^{®}, Envi-Pac-Körper® und dergleichen. Die Dimensionen der Füllkörper wie der durchschnittliche Durchmesser oder die durchschnittliche Dicke oder Länge liegen in der Regel im Bereich von 1 bis 90 mm und insbesondere im Bereich von 5 bis 40 mm. Zur Durchführung des erfindungsgemäßen Verfahrens werden insbesondere z.B. Hiflow-, Pall- oder Super-Ringe mit einem Durchmesser im Bereich von 20 bis 40 mm eingesetzt. Im Allgemeinen liegen die Füllkörper in der Kolonne in Form einer regellosen Schüttung vor. Grundsätzlich sind auch geordnete Schüttungen und/oder Packungen der Füllkörper geeignet.

Zur Durchführung des erfindungsgemäßen Verfahrens wird man im Allgemeinen so vorgehen, dass man den oberen Teil der Kolonne, insbesondere den Kopf der Kolonne kontinuierlich mit der feststoffhaltigen wässrigen Suspension beaufschlagt. Der hierbei eingestellte Stoffstrom hängt unter anderem von den Dimensionen der eingesetzten Kolonne und von den Extraktionsbedingungen, insbesondere der Temperatur ab. Im Allgemeinen wird man die wässrige Suspension mit einer Rate im Bereich von 5 t/(h•m²) bis 40 t/(h•m²) zuführen. Am unteren Teil der Kolonne, insbesondere am Sumpf der Kolonne wird das mit Wasser nicht vollständig mischbare organische Lösungsmittel zugeführt. Das Lösungsmittel ist vor dem Zuführen vorzugsweise mit Wasser gesättigt. Auch hierbei hängt der eingestellte Stoffstrom von den Dimensionen der eingesetzten Kolonne und den Extraktionsbedingungen ab. Im Allgemeinen wird man das organische Lösungsmittel mit einer Rate im Bereich von 5 t/(h•m²) bis 40 t/(h•m²) zuführen. Bei dieser Vorgehensweise bilden sich unter den Extraktionsbedingungen ein erster oberer Bereich mit einer kohärenten organischen Phase im Kopf der Extraktionskolonne und ein weiterer unterer Bereich, in dem Wasser die kohärente Phase bildet. Durch die Zufuhr des organischen Lösungsmittels in dem unteren Bereich der Kolonne wird dieses in der dort befindlichen kohärenten Wasserphase dispergiert, wandert durch die Kolonne als disperse Phase nach oben und vereinigt sich im oberen Bereich der Kolonne zu einer kohärenten organischen Phase. Derjenige Abschnitt der Kolonne, in dem sich die disperse organische Phase zu einer kohärenten organischen Phase vereinigt, wird auch als Phasengrenze bezeichnet. Diese Phasengrenze zwischen der unteren wässrigen und der oberen organischen Phase wird erfindungsgemäß so eingestellt, dass die organische Phase in einem Abschnitt der Kolonne, der wenigstens 50 %, bevorzugt wenigstens 60 % und besonders bevorzugt wenigstens 75 % der Gesamtlänge der Kolonne umfasst, als disperse Phase geführt wird. Unter einer dispersen Phase wird erfingdungsgemäß eine in einer ersten Phase wie einem kontinuierlichen Dispersionsmittel fein verteilt vorliegende weitere Phase verstanden.

Die Grenze zwischen der kohärenten wässrigen und der kohärenten organischen Phase (Phasengrenze) ist vorzugsweise auf gleicher Höhe wie die Füllhöhe der Füllkörper, oberhalb dieser Höhe oder nur unwesentlich darunter, jeweils bezogen auf die gesamte Füllhöhe der Füllkörper, ausgebildet. In einer industriellen Anlage kann die Phasengrenze z.B. im Bereich von 0 bis 2 m oberhalb der Füllhöhe, im Bereich der Füllhöhe selber, oder im Bereich von 0 bis 1 m unterhalb der Füllhöhe ausgebildet sein. Die Füllhöhe selbst wird hierbei vorzugsweise so gewählt, dass die organische Phase in einem Bereich der Kolonne, der wenigstens 75 %, besonders bevorzugt wenigstens 85 % und ganz besonders bevorzugt wenigstens 95 % der Füllkörper umfasst, als disperse Phase geführt wird. Am meisten ist es bevorzugt, die organische Phase im gesamten Bereich der Füllhöhe der Füllkörper als disperse Phase zu führen, so dass die Phasengrenze oberhalb der Füllhöhe liegt. In der Regel wird man die Füllhöhe der Füllkörper so wählen, dass die Kolonne zu wenigstens 50 Vol.-%, insbesondere wenigstens 65 Vol.-% und speziell wenigstens 75 Vol.-%, bezogen auf das Gesamtvolumen der Kolonne, mit Füllkörpern angefüllt ist. Der Begriff der Füllhöhe umfasst hier und im Folgenden sowohl die Schütthöhe als auch die Packungshöhe von in regelloser oder geordneter Schüttung bzw. Packung vorliegenden Füllkörpern. Insoweit wird der Begriff Schütthöhe vorliegend synonym mit dem Begriff Füllhöhe verwendet.

Erfindungsgemäß wird die im oberen Teil der Kolonne zugeführte wässrige Phase somit zunächst als disperse Phase, d.h. fein verteilt in der organischen Phase vorliegend, geführt. An der Phasengrenze tritt dann eine Umkehrung von kohärenter und disperser Phase auf. Die Kolonne ist bis zur Phasengrenze mit der wässrigen Phase geflutet, so dass in diesem Bereich der Kolonne die im unteren Teil der Kolonne durch Zuführung des Lösungsmittels gebildete organische Phase als disperse Phase geführt wird. Es hat sich als vorteilhaft erwiesen, die Phasengrenze etwa in Schütthöhe der Füllkörper einzustellen und die Schütthöhe so zu wählen, dass die wässrige Phase nach Zuführung zunächst in einem Bereich oberhalb der Schütthöhe der Füllkörper als disperse Phase geführt wird. Dieser Bereich liegt üblicherweise in der Größenordnung von wenigen Metern, z.B. im Bereich von etwa 0,5 bis 5 m und insbesondere im Bereich von 1 bis 3 m, jeweils oberhalb der Schütthöhe bzw. Phasengrenze.

Am Kopf der Kolonne, vorteilhafterweise oberhalb des Zulaufs der feststoffhaltigen wässrigen Suspension, wird die feststofffreie organische Phase abgezogen, welche die extrahierte Milchsäure enthält. Die abgezogene feststofffreie organische Phase enthält in der Regel mindestens 5 Gew.-% und insbesondere mindestens 10 Gew.-% Milchsäure, bezogen auf das Gesamtgewicht der abgezogenen organischen Phase.

Am Sumpf der Kolonne kann eine feststoffhaltige wässrige Phase abgezogen werden. Diese enthält als Feststoffe insbesondere Biomasse aus der zur Milchsäureherstellung betriebenen Fermentation und den beim Ansäuern der Fermentationsbrühe ausgefallenen Gips. Die abgezogene feststoffhaltige wässrige Phase enthält vorzugsweise weniger als 2,5 Gew.-%, insbesondere weniger als 1,5 Gew.-% und speziell weniger als 1 Gew.-%, bezogen auf das Gesamtgewicht der erhaltenen feststoffhaltigen wässrigen Phase, an Milchsäure. Hierbei wird der Gehalt an Milchsäure enzymatisch bestimmt, wie z.B. in Ullmann's Encyclopedia of Industrial Chemistry, 5. Aufl., CD-ROM, Enzymes - Enzymes in Analysis and Medicine, 5.3.2 Organic Acids, Lactic Acid, VCH beschrieben. Durch das erfindungsgemäße Extraktionsverfahren können Extraktionsausbeuten von mehr als 90 % und insbesondere von mindestens 92 % der eingesetzten Milchsäure erzielt werden.

Die Extraktion wird kontinuierlich betrieben. Die Extraktion wird in der Regel bei Temperaturen im Bereich von 10 bis 90°C und insbesondere im Bereich von 25 bis 80°C durchgeführt. Die Durchführung des Extraktionsverfahrens bei erhöhter Temperatur hat insbesondere bei Einsatz von Alkanolen als Lösungsmittel Vorteile.

In einer bevorzugten Ausführungsform setzt man daher Alkanole, besonders bevorzugt Butanole und/oder Pentanole und ganz besonders bevorzugt iso-Butanol als Lösungsmittel ein. Dies ist in mehrerer Hinsicht vorteilhaft. Einerseits führt die partielle Wasserlöslichkeit des Alkohols zu einer geringeren Benetzung der hydrophoben Füllkörper und somit zu einer verminderten Koagulation der dispersen Phase an der Oberfläche der Füllkörper. Andererseits wird die Milchsäure bei erhöhter Temperatur bereits in der Extraktionskolonne partiell in die entsprechenden Ester überführt. Dies ist insbesondere dann ein erwünschter Effekt, wenn die extrahierte Milchsäure vollständig oder weitgehend verestert werden soll. Aufgrund dieser Vorteile kann sich insgesamt eine signifikante Einsparung der Destillationskosten ergeben.

Es versteht sich von selber, dass bei einer gegebenenfalls nach der Extraktion durchgeführten Veresterung der Milchsäure, insbesondere mit den als Lösungsmittel verwendeten Alkanolen, ist ein Gehalt von überschüssiger Schwefelsäure in der eingesetzten wässrigen Suspension vorteilhaft, da diese teilweise zusammen mit der Milchsäure extrahiert wird und bei der Veresterung als Katalysator dient. Die vollständige Umsetzung der im Extraktionsschritt noch nicht veresterten Milchsäure kann in bekannter Weise in einer nachfolgenden diskontinuierlichen oder kontinuierlichen Veresterungsstufe erfolgen. Hierfür kann die durch die Extraktion gewonnene organische Phase, die Milchsäure und Milchsäureester enthält, zur Umsetzung der Milchsäure zu Milchsäureester z.B. auf Temperaturen im Bereich von 60 bis 140°C erhitzt werden, wobei man das bei der Umsetzung entstehende Wasser abdestilliert, gegebenenfalls unter Vakuum. Die durch Destillation in reiner Form erhaltenen Milchsäureester sind wertvolle industrielle Zwischenprodukte. Sie können ferner in bekannter Weise wieder zu Milchsäure und Alkohol gespalten werden, so dass auf diese Weise hochreine Milchsäure erhalten wird.

### Beispiel 1 (erfindungsgemäß)

Eine fermentativ hergestellte wässrige Lösung von Calciumlactat wurde mit Schwefelsäure auf einen pH-Wert im Bereich von 1,0 bis 1,5 eingestellt. Die Suspension mit einem Gehalt von 11 Gew.-% an Milchsäure und einem Feststoffanteil von 8 Gew.-% wurde auf eine Temperatur von 70°C erhitzt. Der Kopf einer Füllkörperkolonne (Länge 18 m, Durchmesser 0,6 m, regellose Schüttung aus Polypropylen-Pallringen mit 30 mm Durchmesser, Schütthöhe 15 m) wurde kontinuierlich mit 2,5 t/h dieser Suspension beaufschlagt. In den Sumpf der Kolonne wurden gleichzeitig im Gegenstrom 3,1 t/h mit Wasser gesättigtes Isobutanol geleitet. Die Phasengrenze Isobutanol/Wasser wurde in Höhe der Schütthöhe der Füllkörper eingestellt. Auf diese Weise wurde an der Phasengrenze eine Umkehrung von kontinuierlicher und disperser Phase erreicht. Zunächst wurde die wässrige Phase als disperse Phase geführt (ca. 1 bis 2 m). Bis zur Phasengrenze war die Kolonne von der Wasserphase geflutet, so dass die am Kolonnensumpf zugegebene Isobutanolphase in diesem unteren Bereich als disperse Phase geführt wurde. Die mit Isobutanol gesättigte wässrige Phase einschließlich des Feststoffgehalts an Gips und Biomasse wurde am Sumpf der Kolonne abgezogen. Am Kolonnenkopf lief der feststofffreie, milchsäurehaltige Extrakt ab. Dieser stand zur destillativen Aufarbeitung zur Verfügung.

Die am Sumpf der Kolonne erhaltene wässrige Phase enthielt 0,5 bis 1,0 Gew.-% an Milchsäure, bezogen auf das Gesamtgewicht der am Sumpf der Kolonne erhaltenen wässrigen Phase, ermittelt durch enzymatische Bestimmung. Dies entspricht einer Extraktionsausbeute von 92 bis 96 % Milchsäure, bezogen auf die eingesetzte Milchsäure. Bei Verwendung von Füllkörpern aus Polyvinylidenfluorid wurden ähnliche Ergebnisse erzielt.

### Beispiel 2 (gemäß EP-A-0 159 585)

Die im obigen Beispiel 1 beschriebene Kolonne wurde mit der gleichen Belastung so betrieben, dass sich die Phasengrenze Isobutanol/Wasser im Sumpf der Kolonne befand. Somit wurde die zu extrahierende wässrige Phase im Wesentlichen als disperse Phase geführt. Wie in Beispiel 1 wurde am Sumpf der Kolonne die gipshaltige Wasserphase und am Kolonnenkopf die Isobutanolphase abgezogen.

Die am Sumpf der Kolonne erhaltene wässrige Phase enthielt 3,1 bis 3,5 Gew.-% an Milchsäure, bezogen auf das Gesamtgewicht der am Sumpf der Kolonne erhaltenen wässrigen Phase, ermittelt durch enzymatische Bestimmung. Dies entspricht einer Extraktionsausbeute von 72 bis 75 % Milchsäure, bezogen auf die eingesetzte Milchsäure.

## Patentansprüche

1. Verfahren zur Extraktion von Milchsäure aus einer feststoffhaltigen wässrigen Suspension, bei dem man die feststoffhaltige wässrige Suspension in einer mit Füllkörpern versehenen Kolonne, wobei die Oberflächen der Füllkörper aus hydrophobem Material bestehen, im Gegenstrom mit einem mit Wasser nicht vollständig mischbaren organischen Lösungsmittel unter Ausbildung einer wässrigen und einer organischen Phase so in Kontakt bringt, dass die organische Phase zumindest in einem Abschnitt der Kolonne, der Füllkörper umfasst, als disperse Phase geführt wird.

2. Verfahren nach Anspruch 1, wobei die Füllhöhe der Füllkörper so gewählt ist, dass wenigstens 50 Vol.-%, bezogen auf das Gesamtvolumen der Kolonne, mit Füllkörpern gefüllt sind.

3. Verfahren nach Anspruch 1 oder 2, wobei die organische Phase in einem Abschnitt der Kolonne, der wenigstens 50 % der Füllhöhe der Füllkörper umfasst, als disperse Phase geführt wird.

4. Verfahren nach einem der vorhergehenden Ansprüche, wobei der Kopf der Kolonne kontinuierlich mit der feststoffhaltigen Suspension beaufschlagt wird und am Sumpf der Kolonne das mit Wasser nicht vollständig mischbare organische Lösungsmittel zugeführt wird.

5. Verfahren nach Anspruch 4, wobei man am Kopf der Kolonne oberhalb des Zulaufs der feststoffhaltigen Suspension eine feststofffreie organische Phase und am Sumpf der Kolonne eine feststoffhaltige wässrige Phase abzieht.

6. Verfahren nach einem der vorhergehenden Ansprüche, wobei sich im oberen Bereich der Kolonne eine kohärente organische Phase und im unteren Bereich der Kolonne eine kohärente wässrige Phase ausbildet und die Grenze zwischen der kohärenten wässrigen und der kohärenten organischen Phase auf gleicher Höhe wie die Füllhöhe der Füllkörper, oberhalb dieser Höhe oder nur unwesentlich darunter ausgebildet ist.

7. Verfahren nach einem der vorhergehenden Ansprüche, wobei die feststoffhaltige wässrige Suspension durch Ansäuern einer fermentativ hergestellten wässrigen Lösung von Calciumlactat mit Schwefelsäure erhalten wurde.

8. Verfahren nach einem der vorhergehenden Ansprüche, wobei das mit Wasser nicht vollständig mischbare organische Lösungsmittel eine unter Alkanolen, Ketonen, Ethern und Estern ausgewählte aliphatische oder cycloaliphatische sauerstoffhaltige Verbindung mit mindestens 4 C-Atomen oder ein Gemisch davon ist.

9. Verfahren nach Anspruch 8, wobei man als mit Wasser nicht vollständig mischbares organisches Lösungsmittel ein Alkanol mit mindestens 4 C-Atomen einsetzt.

10. Verfahren nach Anspruch 9, wobei man Isobutanol als Alkanol einsetzt.

11. Verfahren nach einem der Ansprüche 9 oder 10, wobei die durch die Extraktion gewonnene organische Phase, die Milchsäure und Milchsäureester enthält, zur Umsetzung der Milchsäure zu Milchsäureester auf Temperaturen im Bereich von 60 bis 140°C erhitzt, wobei man das bei der Umsetzung entstehende Wasser abdestilliert.

12. Verfahren nach einem der vorhergehenden Ansprüche, wobei die Extraktion bei Temperaturen im Bereich von 10 bis 90°C durchgeführt wird.

13. Verfahren nach einem der vorhergehenden Ansprüche, wobei man nach der Extraktion eine feststoffhaltige wässrige Phase erhält, die weniger als 2,5 Gew.-%, bezogen auf das Gesamtgewicht der erhaltenen feststoffhaltigen wässrigen Phase, Milchsäure enthält.

## Claims

1. A process for the extraction of lactic acid from a solids-comprising aqueous suspension, in which the solids-comprising aqueous suspension is brought into contact in countercurrent with an organic solvent which is not completely miscible with water to form an aqueous phase and an organic phase in a column provided with packing elements whose surfaces comprise hydrophobic material in such a way that the organic phase is conveyed as disperse phase at least in one section of the column comprising packing elements.

2. The process according to claim 1, wherein the fill height of the packing elements is selected so that at least 50% by volume of the total volume of the column is filled with packing elements.

3. The process according to claim 1 or 2, wherein the organic phase is conveyed as disperse phase in a section of the column which comprises at least 50% of the fill height of the packing elements.

4. The process according to any of the preceding claims, wherein the solids-comprising suspension is fed in continuously at the top of the column and the organic solvent which is not completely miscible with water is introduced at the bottom of the column.

5. The process according to claim 4, wherein a solids-free organic phase is taken off at the top of the column above the point at which the solids-comprising suspension is fed in and a solids-comprising aqueous phase is taken off at the bottom of the column.

6. The process according to any of the preceding claims, wherein a continuous organic phase is formed in the upper region of the column and a continuous aqueous phase is formed in the lower region of the column and the boundary between the continuous aqueous phase and the continuous organic phase is formed at the same height as the fill height of the packing elements, above this height or only insignificantly below it.

7. The process according to any of the preceding claims, wherein the solids-comprising aqueous suspension has been obtained by acidification of an aqueous solution of calcium lactate prepared by fermentation with sulfuric acid.

8. The process according to any of the preceding claims, wherein the organic solvent which is not completely miscible with water is an aliphatic or cycloaliphatic oxygen-comprising compound having at least 4 carbon atoms selected from among alkanols, ketones, ethers and esters or a mixture thereof.

9. The process according to claim 8, wherein an alkanol having at least 4 carbon atoms is used as organic solvent which is not completely miscible with water.

10. The process according to claim 9, wherein isobutanol is used as alkanol.

11. The process according to either claim 9 or 10, wherein the organic phase obtained by means of the extraction, which comprises lactic acid and lactic esters, is heated to temperatures in the range from 60 to 140°C to convert the lactic acid into lactic esters, with the water formed in the reaction being distilled off.

12. The process according to any of the preceding claims, wherein the extraction is carried out at temperatures in the range from 10 to 90°C.

13. The process according to any of the preceding claims, wherein a solids-comprising aqueous phase comprising less than 2.5% by weight, based on the total weight of the solids-comprising aqueous phase obtained, of lactic acid is obtained after the extraction.

## Revendications

1. Procédé pour l'extraction d'acide lactique à partir d'une suspension aqueuse contenant des matières solides, dans lequel on met en contact à contre-courant avec des solvants organiques non totalement miscibles à l'eau la suspension aqueuse contenant des matières solides, dans une colonne munie d'éléments de garnissage, les surfaces des éléments de garnissage étant constituées d'un matériau hydrophobe, avec formation d'une phase aqueuse et d'une phase organique, de manière que la phase organique soit conduite en tant que phase dispersée au moins dans une section de la colonne qui comporte des éléments de garnissage.

2. Procédé selon la revendication 1, dans lequel la hauteur de remplissage des éléments de garnissage est choisie de manière qu'au moins 50 % en volume, par rapport au volume total de la colonne, soient remplis avec des éléments de garnissage.

3. Procédé selon la revendication 1 ou 2, dans lequel la phase organique est conduite en tant que phase dispersée dans une section de la colonne qui comprend au moins 50 % de la hauteur de remplissage des éléments de garnissage.

4. Procédé selon l'une quelconque des revendications précédentes, dans lequel la tête de la colonne est chargée en continu avec la suspension contenant des matières solides et au pied de la colonne est envoyé le solvant organique non totalement miscible à l'eau.

5. Procédé selon la revendication 4, dans lequel une phase organique exempte de matières solides est évacuée à la tête de la colonne au-dessus de l'arrivée de la suspension contenant des matières solides et une phase aqueuse contenant des matières solides est évacuée au pied de la colonne.

6. Procédé selon l'une quelconque des revendications précédentes, dans lequel une phase organique cohérente est formée dans la zone supérieure de la colonne et une phase aqueuse cohérente est formée dans la zone inférieure de la colonne et la frontière entre la phase aqueuse cohérente et la phase organique cohérente est formée à la même hauteur que la hauteur de remplissage des éléments de garnissage, au-dessus de cette hauteur ou seulement très légèrement au-dessous de celle-ci.

7. Procédé selon l'une quelconque des revendications précédentes, dans lequel la suspension aqueuse contenant des matières solides a été obtenue par acidification avec de l'acide sulfurique d'une solution aqueuse de lactate de calcium produite par fermentation.

8. Procédé selon l'une quelconque des revendications précédentes, dans lequel le solvant organique non totalement miscible à l'eau est un composé aliphatique ou cycloaliphatique oxygéné ayant au moins 4 atomes de carbone, choisi parmi des alcanols, des cétones, des éthers et des esters.

9. Procédé selon la revendication 8, dans lequel on utilise comme solvant organique non totalement miscible à l'eau un alcanol ayant au moins 4 atomes de carbone.

10. Procédé selon la revendication 9, dans lequel on utilise comme alcanol l'isobutanol.

11. Procédé selon la revendication 9 ou 10, dans lequel la phase organique obtenue par l'extraction, qui contient de l'acide lactique et des esters d'acide lactique, est chauffée à des températures dans la plage de 60 à 140 °C, pour la conversion de l'acide lactique en ester d'acide lactique, avec élimination par distillation de l'eau formée lors de la réaction.

12. Procédé selon l'une quelconque des revendications précédentes, dans lequel l'extraction est effectuée à des températures dans la plage de 10 à 90 °C.

13. Procédé selon l'une quelconque des revendications précédentes, dans lequel on obtient après l'extraction une phase aqueuse, contenant des matières solides, qui contient moins de 2,5 % en poids, par rapport au poids total de la phase aqueuse obtenue, contenant des matières solides, d'acide lactique.
